# EUROPEAN PATENT SPECIFICATION

(11) **EP 2 205 620 B1**
(45) Date of publication and mention of the grant of the patent: **13.04.2011**
(21) Application number: 08841744.9
(22) Date of filing: 23.10.2008
(51) Int. Cl.: C07J 41/00, A61K 31/57, A61P 17/06

(54) **Amorphous form of (11beta,16alpha)-9-fluoro-11-hydroxy-16,17-[(1-methylethyliden)bis(oxy)]-21-[[4- [(nitrooxy)methyl]benzoyl]oxy]-pregna-1,4-dien-3,20-dione**
Amorphe Form von (11beta,16alpha)-9-Fluoro-11-hydroxy-16,17-[(1-methylethyliden)bis(oxy)]-21-[[4- [(nitrooxy)methyl]benzoyl]oxy]-pregna-1,4-dien-3,20-dion
Forme amorphe de (11bêta,16alpha)-9-fluoro-11-hydroxy-16,17-[(1-méthyléthylidèn)bis(oxy)]-21-[[4- [(nitrooxy)méthyl]benzoyl]oxy]-pregna-1,4-dièn-3,20-dione

(30) Priority: 25.10.2007 ES 200702796
(43) Date of publication of application: 14.07.2010
(73) Proprietor: Ferrer Internacional, S.A., 08028 Barcelona (ES); Nicox S.A., 06560 Sophia Antipolis - Valbonne (FR)
(72) Inventor: ANGLADA, Luis, E-08009 Barcelona (ES); ALBET, Carlos, 08906 L'HOSPITALET DE LLOBREGAT (Barcelona) (ES); GUGLIETTA, Antonio, E-08750 Molins De Rei (ES)
(74) Representative: Reitstötter - Kinzebach
(86) International application number: PCT/EP2008/064387
(87) International publication number: WO 2009/053439

(56) References cited:
- WO-A-03/064443
- WO-A-2007/025632

## Description

### Field of the art

The invention relates to the technical field of anti-inflammatory compounds, specifically those of a steroid nature, in particular to a new amorphous form of a nitrooxy derivative of a corticosteroid, its pharmaceutical formulations and its use in the treatment or prevention of diseases or symptoms of the skin or mucous membranes.

### State of the art

Most of the diseases or symptoms of the skin or mucous membranes are caused by the swelling caused by inflammatory agents, such as, by way of example of a non-limiting nature, malignant bacterial, fungal, viral, parasitic, autoimmune, allergic, hormonal and/or inflammatory agents. The most common diseases or symptoms of the skin or the mucous membranes include, by way of example of a non-limiting nature, skin scabs, atopic dermatitis, contact dermatitis, seborrheic dermatitis, dermatosis, eczema, epidermolysis bullosa, erythemas, erosions, skin flakes, exudation, inflammation, lichen planus, red lichen, papulation, pruritus, rash due to diapers, tinea cruris, psoriasis and warts. Dermatitides and eczema occur as a result of inflammatory processes involving the upper dermis and the epidermis. When the eczema develops, the keratinocytes in the epidermis dilate and liquid accumulates inside them in a process known as spongiosis. In chronic forms of eczema or dermatitis the main change includes bulging of the epidermis, which causes itching, roughness and skin flakes on the surface of the skin. The loss of water from the skin causes swelling of the stratum corneum, which translates into cracked and painful skin. Moreover, dermatitides can be classified into contact dermatitis (allergic or non-allergic), atopic dermatitis and seborrheic dermatitis. Non-allergic contact dermatitis occurs as a response to skin irritants, such as acids, alkalis, oils, detergents and solvents.

Atopic dermatitis is a recurrent and/or chronic swelling of the skin of immunological origin that is triggered by a wide variety of common antigens. It is characterised by an eruption with intense pruritus and eczema, often erythematous. It appears mostly during childhood, and the body parts affected are usually the face, neck, upper trunk, wrists, hands and skinfolds. It is pretty widespread worldwide in children, reaching up to 30% depending on the country, and is somewhat more minoritary in adults. It is a disease that seriously affects the patient's quality of life and their family environment.

Allergic contact dermatitis occurs as a result of the sensitisation to repeated exposure to an antigen. Allergic contact dermatitis occurs in areas of the skin that have been in contact with the antigen.

Seborrheic dermatitis affects the scalp and other areas that are covered by hair, the face, areas with skinfolds, and as a result of swelling induced by yeasts or bacteria. Most of the population suffers from dandruff, which is a mild form of seborrheic dermatitis. Psoriasis is a dominant autosomal inflammatory disease characterised by a proliferation of keratinocytes the proliferation of which leads to the formation of squamous plates on, for example, knees, elbows and buttocks. They are aesthetically unpleasing and cause discomfort to the person affected.

Skin diseases are usually treated with creams, gels or ointments containing steroidal agents and/or antibacterial agents and/or antifungal agents.

Topical corticosteroids are a powerful tool for the treatment of skin diseases. However, in clinical practice, the use of superpowerful topical steroids is typically limited to only two weeks, since these are usually associated to side-effects such as skin atrophy, burning, itching, irritation, dryness, folliculitis, hypertrichosis, acne, hypopigmentation, perioral dermatitis, allergic contact dermatitis, skin maceration and secondary infections.

Although the topical administration of corticosteroids minimises the side effects as compared to systemic administration, the active ingredients may pass into the bloodstream, thus becoming systemically active. Systemic absorption of corticosteroids may cause a reversible suppression of the hypothalamic-pituitary-adrenal axis (HPA), symptoms such as Cushing's syndrome, hyperglycaemia, effects on bone growth in children and on bone density in the elderly, eye complications (formation of cataracts and glaucoma) and skin atrophy. Moreover, the use of topical corticosteroids may also cause tachyphylaxis.

Despite modern glucocorticoids being much safer than those originally introduced, the production of new molecules and formulations having improved clinical efficacy and less side effects is still under research. Several products have been developed in order to increase the efficiency and/or efficacy of topical agents, although such products have had limited success. A large variety of topical formulations have thus been developed, such as creams, lotions, gels and the like, trying to increase the efficiency of active ingredient release. However, despite the direct and localised application of the dermatological agent to the skin surface, very few topical formulations have provided a complete solution, since typically only partial improvements have been achieved even with supposedly optimal formulations, and the skin lesions have often persisted without treatment times having noticeably shortened.

US patent 4335121 describes the S-fluoromethyl ester of 6α, 9α-difluoro-17α-(1-oxopropoxy)-11 1β-hydroxy-16α-methyl-3-oxo-androsta-1,4-dien-17-0-carbothioic acid (known by its generic name fluticasone propionate) and its derivatives. These compounds have good anti-inflammatory activity, especially in topical applications.

Patent document EP 929565 describes the nitrooxy esters of corticosteroids, amongst the uses of which is the treatment of skin diseases; the patent document specifically describes nitrooxy esters of corticosteroids in which the nitrooxy group is covalently bound by means of an alkyl chain to the glucocorticoid half. The document states that these nitro derivatives of steroidal compounds, after systemic administration, show greater efficacy and better systemic tolerance, such as better gastric tolerance and reduced cardiovascular side-effects as compared to their originating compounds.

Patent application WO 03064443 describes nitrooxy derivatives of corticosteroids in which the nitrooxy group is covalently bound by means of a connector WOR the glucocorticoid half, said connector having an aromatic ring or heteroaryl. The document reports that these nitrooxy derivatives of steroidal compounds show improved pharmacological activity and less side effects compared to their precursor compounds.

Patent application WO 0061604 describes nitrooxy derivatives of corticosteroids in which the nitrooxy group is covalently bound by means of an "antioxidant half' to the glucocorticoid half, the "antioxidant halves" being fragments of compounds capable of preventing the production of selected free radicals based on tests described in the application. The document reports that these compounds can be used for the treatment of pathologies associated with a situation of oxidizing stress in which the corresponding precursor compounds show lower activity or greater toxicity.

Said documents do not describe the activity of nitrooxy derivatives of corticosteroids after topical administration and does not specifically provide any information on having studied the local tolerance of the compounds.

Patent application WO 9734871 describes nitrosated or nitrosylated steroids and their use in the treatment of respiratory diseases, specifically describing the activity of 9-fluoro-11β-hydroxy-16α,17α-[(1-methylethyliden)bis(oxy)]pregna-1,4-dien-3,20-dione-21 (4-nitrooxy)-butanoate in a lung model of allergic asthma and pulmonary inflammation. The application does not mention the use of these compounds in the treatment of skin diseases.

Hyun E. et al., British Journal of Pharmacology (2004) 143, 618-625, refers to the study of hydrocortisone 21-[4'-(nitrooxymethyl)benzoate] activity in an acute dermatitis model, evaluating in this study the formation of the oedema and the recruiting of leukocytes, the results demonstrating that the compound has greater anti-inflammatory activity than its precursor hydrocortisone. The document does not provide any information on the effect of the compound on the skin after extended treatment. Moreover, the experimental model described by Hyun E. et al. is not predictive for other dermatological diseases.

Patent application WO 2007025632 describes nitrooxy derivatives of steroidal compounds, the topical formulations that contain them and their use in the treatment of diseases of the skin or the mucous membranes, showing improved pharmacological activity and increased local tolerance. One of the compounds specifically claimed is (11β,16α)-9-fluoro-11-hydroxy-16,17-[(1-methylethyliden)bis(oxy)]-21-[[4-[(nitrooxy)methyl]benzoyl]oxy]-pregna-1,4-dien-3,20-dione.

### Brief description of the drawings

Figures 1 and 2 show X-ray powder diffraction curves of compound (I) in the amorphous form and the crystalline form respectively. Intensity, on the y-axis, is expressed in cps. The x-axis corresponds to the 2θ angle.
Figures 3 and 4 show the Fourier transform Raman Spectrum of compound (I) in the amorphous form and the crystalline form respectively. The y-axis corresponds to intensity. The x-axis corresponds to wave numbers, expressed in cm⁻¹.
Figures 5 and 6 show the Differential Scanning Calorimetry for compound (I) in the amorphous form and the crystalline form respectively. The y-axis corresponds to heat flows, expressed in mW. The x-axis corresponds to temperature, expressed in °C.

### Detailed description of the invention

The present invention relates to an amorphous form of an anti-inflammatory steroidal compound corresponding chemically to (11β,16β)-9-fluoro-11-hydroxy-16,17-[(1-methylethyliden)bis(oxy)]-21-[[4-[(nitrooxy)methyl]penzoyl]oxy]-pregna-1,4-dien-3,20-dione, as well as its preparation procedures, its use as a therapeutically active agent and the pharmaceutical compositions comprising the new form.

Throughout this application the term "compound (I)" relates to (11β,16α)-9-fluoro-11-hydroxy-16,17-[(1-methylethyliden)bis(oxy)]-21-[[4-[(nitrooxy)methyl]benzoyl]oxy]-pregna-1,4-dien-3,20-dione.

Patent application WO 2007025632 describes compound (I) and its preparation. Its structural formula is:

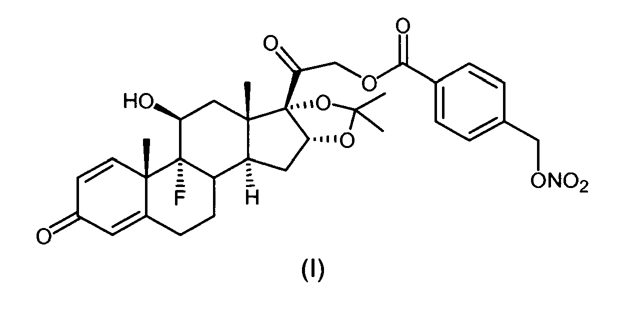

The compound (I) has an important topical anti-inflammatory activity. It is clinically useful in the treatment or prevention of several diseases or symptoms of the skin or the mucous membranes, such as skin scabs, atopic dermatitis, contact dermatitis, seborrheic dermatitis, dermatosis, eczema, epidermolysis bullosa, erythemas, erosions, skin flakes, exudation, inflammation, lichen planus, red lichen, papulation, pruritus, rash due to diapers, tinea cruris, psoriasis and warts. It is typically applied as creams, lotions, ointments, solutions for pulverisation or the like.

Since the presentations of the formulations of compound (I) are generally liquid or semiliquid and the compound as obtained in the aforementioned application is a crystalline material that is difficult to handle in the manufacture of said preparations, the need arises to obtaining compound (I) in a form that is easier to handle for this manufacture. Therefore, during the milling of the crystals of compound (I) in an Agatha mortar, it was casually discovered that the crystals partially transformed into an amorphous material. The amorphous material is extremely advantageous for the manufacture of liquid and semiliquid preparations and constitutes a solution to the problem of the use of compound (I) in crystalline form when manufacturing liquid or semiliquid preparations.

The present invention refers to compound (I) in amorphous form in a main embodiment thereof.

The conventional procedures for obtaining amorphous substances comprise the fusion of the corresponding crystalline substances and the fast cooling of the fused materials. However, such procedures are usually limited to a laboratory scale and are impracticable and scarcely suitable to an industrial scale. Similarly, the procedure described above of milling in an Agatha mortar does not quantitatively provide the amorphous form, and is neither therefore a suitable procedure for industrialisation.

Therefore, in another embodiment, the present invention provides a procedure for the preparation of the amorphous form of compound (I) comprising:
(i) dissolving compound (I) in dioxane;
(ii) filtering the solution; and
(iii) recovering the resulting amorphous compound (I) by freeze-drying at a temperature of -5 to 5°C.

In a preferred embodiment of the present invention, the amorphous compound (I) obtained in stage (iii) is dried by freeze-drying at a temperature of -2 to 2°C.

In a preferred embodiment of the present invention, the amorphous compound (I) obtained in stage (iii) is dried by freeze-drying at a temperature of 0°C.

In another embodiment of the present invention compound (I) in amorphous form is used for the preparation of a topical drug.

In another embodiment of the present invention compound (I) in amorphous form is used for the preparation of a drug in the form of creams, lotions, ointments, solutions for pulverisation and the like.

In another embodiment, the present invention relates to a pharmaceutical formulation comprising compound (I) in amorphous form to be used in the treatment or prevention of several diseases or symptoms of the skin or the mucous membranes, comprising skin scabs, atopic dermatitis, contact dermatitis, seborrheic dermatitis, dermatosis, eczema, epidermolysis bullosa, erythemas, erosions, skin flakes, exudation, inflammation, lichen planus, red lichen, papulation, pruritus, rash due to diapers, tinea cruris, psoriasis and warts.

In another embodiment, the present invention relates to the use of compound (I) in amorphous form in the manufacture of a topical drug comprising creams, lotions, ointments and solutions for pulverisation to be used in the treatment or prevention of several diseases or symptoms of the skin or of the mucous membranes, comprising skin scabs, atopic dermatitis, contact dermatitis, seborrheic dermatitis, dermatosis, eczema, epidermolysis bullosa, erythemas, erosions, skin flakes, exudation, inflammation, lichen planus, red lichen, papulation, pruritus, rash due to diapers, tinea cruris, psoriasis and warts.

The preferred pharmaceutical forms include creams, lotions, ointments and solutions for pulverisation. Said pharmaceutical forms are prepared according to well known procedures in the art.

The proportions of compound (I) in the topical formulations of the present invention depend on a specific type of formulation to be prepared, usually ranging from 0.001 to 12% by weight. However, for most preparations, the most advantageous proportions usually range from 0.001 to 1%, more preferably from 0.01 to 0.5% and especially approximately from 0.025 to 0.1 %. Several pharmaceutically acceptable inactive ingredients may also be present in the different formulations. Said ingredients are: one or more solvents such as several alcohols including, but limited to, ethanol, propylene glycol, triacetin, hexylene glycol and combinations thereof; suitable occlusive agents that may be present in the topical formulations and that include but are not limited to petroleum jelly, microcrystalline wax, dimethicone, beeswax, mineral oil, squalane, liquid paraffin, shea butter, carnauba wax, SEPIGEL (a mixture of isoparaffin, polyacrylamide and lauryl alcohol 7 OE) and combinations thereof; surfactants such as, but not limited to CETOMACROGOL 1000, (Crodor, Inc.), glycerin stearates, polyoxyethylene stearates, a mixture of glycerin stearate and PEG-100 Stearate (such as ARLACEL 165), polysorbate 40, polysorbate 60, polysorbate 80, CETETH-20, sorbitan monopalmitate, sorbitan monostearate, sorbitan monooleate and combinations thereof. Many other inactive ingredients may also be present in topical formulations. These can be carriers (such as water or mineral oils), skin conditioners (such as lanolin, glycerin, cholesterol, cetostearyl alcohol, dimethicone, PEG 100, PEG 200, PEG 300, PEG 400 or isopropyl myristate), buffers (such as citrate/citric acid, sodium phosphate dibasic/citric acid, or sodium phosphate monobasic/citric acid) or preservatives (such as imidurea, methylparaben or propylparaben).

### Embodiments of the invention

The present invention is additionally illustrated by the following example, which does not intend to limit its scope.

### Example 1: Preparation of the amorphous form of (11β,16α)-9-fluoro-11-hydroxy-16,17-[(1-methylethyliden)bis(oxy)]-21-[[4-[(nitrooxy)methyl]benzoyl]oxy]-pregna-1,4-dien-3,20-dione

160 mg of (11β,16α)-9-fluoro-11-hydroxy-16,17-[(1-methylethyliden)bis(oxy)]-21-[[4-[(nitrooxy)methyl]benzoyl]oxy]-pregna-1,4-dien-3,20-dione were dissolved in 10 ml of dioxane in a 25 mL flask. The solution was filtered, the material was recovered by freeze-drying at 0°C, and was then kept refrigerated at the same temperature.

### CHARACTERISTICS OF THE AMORPHOUS FORM

The amorphous form of compound (I) was characterized using the following procedures.

### Instruments and experimental conditions

*X-ray powder diffraction:* Bruker D8 Advance. Radiation Cu Kα (λ = 1.5418 Å); tube power 35kV/45mA; VANTEC1 detector; interval size 2θ 0.017°, 105±5 s per interval, scanning interval 2θ 2°-50°. The sample slides used were in single glass silica, of 12 mm in diameter.
*FT-Raman spectroscopy:* Bruker RFS100. Nd:YAG excited at 1064 nm, laser power 100 mW, Ge-detector, 64 scans, interval 50-3500 cm⁻¹, resolution 2 cm⁻¹. An aluminium sample slide was used.
*Differential Scanning Calorimetry:* Perkin Elmer DSC 7. The crucibles used were in gold.

### Characteristics of the amorphous form

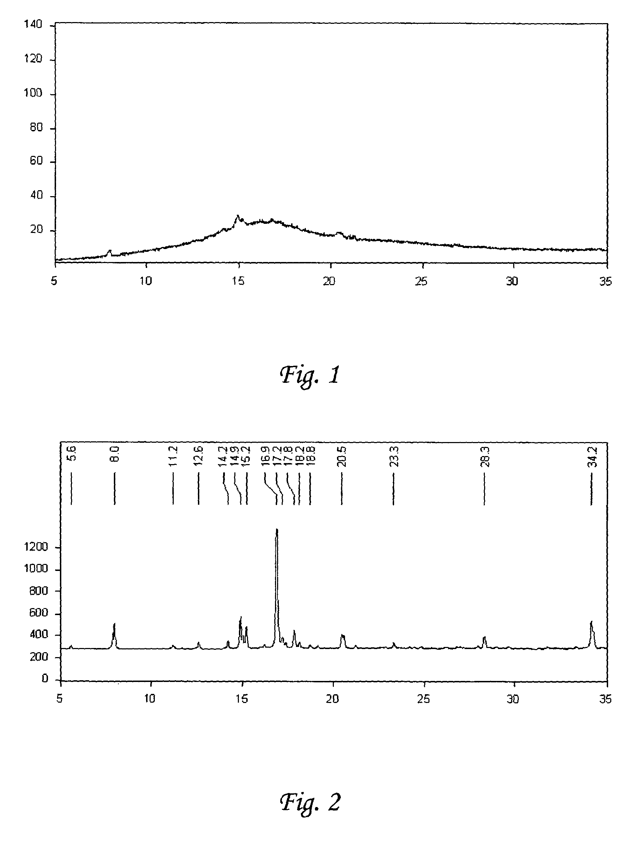

The X-ray powder diffraction pattern for compound (I) in amorphous form shows a broad halo consisting in a slight increase in the base line, characteristic of an amorphous material (Fig. 1). On the other hand, the crystalline form shows the most intense peaks at 2θ to 8.0°, 14.9°, 15.2° and 16.9° (Fig. 2).

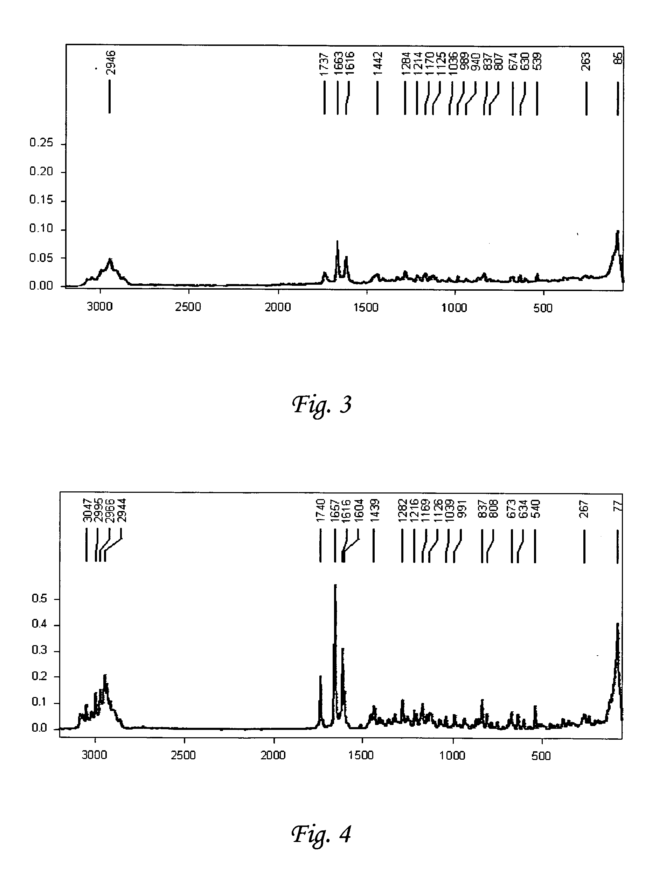

The Raman spectrum for compound (I) in amorphous form is characterised by a significant broadening of the peaks (Fig. 3). The positions of the peaks are the same as those for the crystalline form, but part of the fine structure has been lost. The most intense peaks of the crystalline form originate from the C=O and C=C vibrations, with frequencies of 1740 cm⁻¹, 1657 cm⁻¹, 1616 cm⁻¹ and 1604 cm⁻¹; there is a large number of well resolved peaks in the C-H region (Fig. 4).

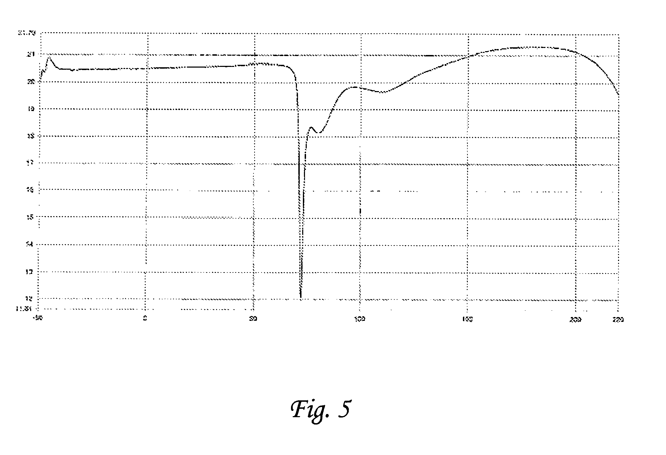

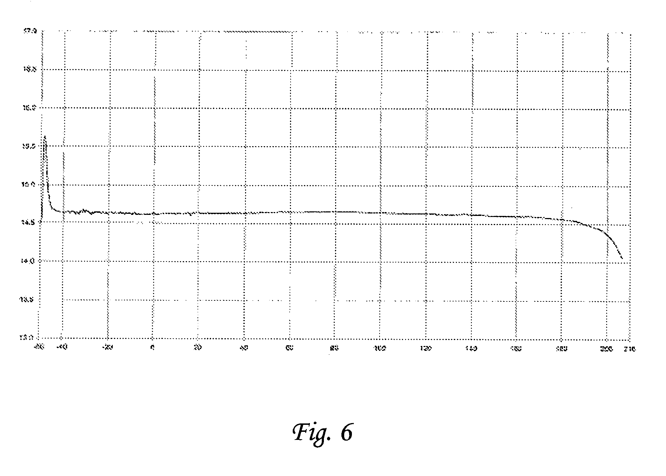

The Differential Scanning Calorimetry for compound (I) in amorphous form shows a poorly resolved glass transition close to 40°C with a ΔCp = 0.23 J/(g K). The glass transition is followed by recrystallisation with a peak exothermicity at 71°C and a recrystallisation enthalpy of 150 J/g (Fig. 5). On the other hand, the material in crystalline form does not show any event until it starts to decompose, at approximately 200°C (Fig. 6).

## Claims

1. An amorphous form of a steroidal anti-inflammatory compound of formula (1):

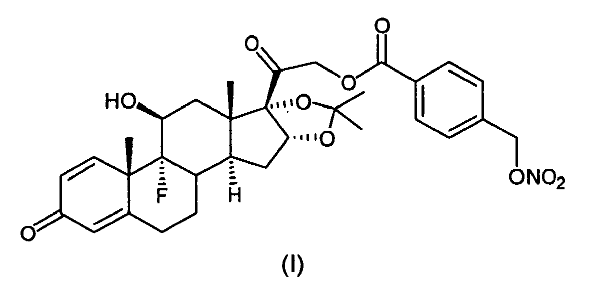

2. A procedure for the preparation of an amorphous form according to claim 1, comprising:
(i) dissolving compound (I) in dioxane;
(ii) filtering the solution; and
(iii) recovering the resulting amorphous compound (I) by freeze-drying at a temperature of -5 to 5°C.

3. The procedure according to claim 2 wherein the compound (I) amorphous of stage (iii) is freeze-dried at a temperature of -2 to 2°C.

4. The procedure according to claim 3 wherein the compound (I) amorphous of stage (iii) is freeze-dried at a temperature of 0°C.

5. A pharmaceutical formulation comprising the amorphous form according to claim 1 and one or more pharmaceutically acceptable excipients.

6. The formulation of claim 5 in the form of a cream, lotion, ointment and solution for pulverisation.

7. The amorphous form of claim 1 for use in the treatment or prevention of diseases or symptoms of the skin or of the mucous membranes.

8. The amorphous form of claim 7 for use in the treatment or prevention of skin scabs, atopic dermatitis, contact dermatitis, seborrheic dermatitis, dermatosis, eczema, epidermolysis bullosa, erythemas, erosions, skin flakes, exudation, inflammation, lichen planus, red lichen, papulation, pruritus, rash due to diapers, tinea cruris, psoriasis or warts.

9. The amorphous form of claim 7 or 8 which is used as cream, lotion, ointment and solution for pulverisation.

## Patentansprüche

1. Amorphe Form einer steroidalen antiinflammatorischen Verbindung der Formel (1):

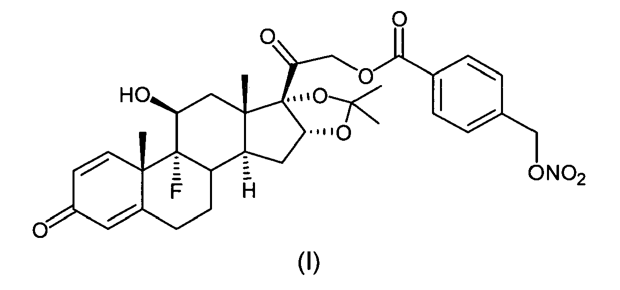

2. Verfahren zur Herstellung einer amorphen Form nach Anspruch 1, umfassend:
(i) Lösen der Verbindung (1) in Dioxan;
(ii) Filtrieren der Lösung; und
(iii) Gewinnen der resultierenden amorphen Verbindung (1) durch Gefriertrocknen bei einer Temperatur von -5 bis 5°C.

3. Verfahren nach Anspruch 2, wobei die amorphe Verbindung (1) der Stufe (iii) bei einer Temperatur von -2 bis 2°C gefriergetrocknet wird.

4. Verfahren nach Anspruch 3, wobei die amorphe Verbindung (1) der Stufe (iii) bei einer Temperatur von 0°C gefriergetrocknet wird.

5. Pharmazeutische Formulierung, umfassend die amorphe Form nach Anspruch 1 und einen oder mehrere pharmazeutisch akzeptable Exzipienten.

6. Formulierung nach Anspruch 5 in Form einer Creme, Lotion, Salbe und Lösung zum Zerstäuben.

7. Amorphe Form nach Anspruch 1 zur Verwendung bei der Behandlung oder Prävention von Erkrankungen oder Symptomen der Haut oder der Schleimhaut.

8. Amorphe Form nach Anspruch 7 zur Verwendung bei der Behandlung oder Prävention von Hautschorf, atopischer Dermatitis, Kontaktdermatitis, seborrhoischer Dermatitis, Dermatose, Ekzemen, Epidermolysis bullosa, Erythemen, Erosionen, Hautabschuppungen, Exsudation, Entzündungen, Lichen ruber planus, Rotflechte, Papelbildung, Pruritus, durch Windeln verursachten Ausschlag, Tinea cruris, Psoriasis oder Warzen.

9. Verwendung der amorphen Form nach Anspruch 7 oder 8 als Creme, Lotion, Salbe und Lösung zum Zerstäuben.

## Revendications

1. Forme amorphe d'un composé anti-inflammatoire stéroïdien de la formule (1) :

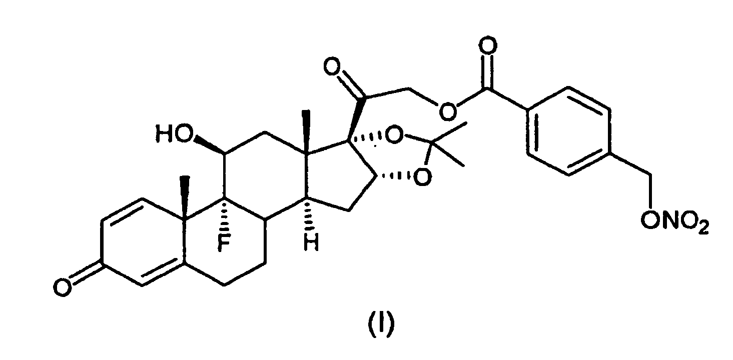

2. Procédure destinée à la préparation d'une forme amorphe selon la revendication 1, comprenant le fait :
(i) de dissoudre le composé (1) dans du dioxane ;
(ii) de filtrer la solution ; et
(iii) de restaurer le composé amorphe résultant (1) par lyophilisation à une température de -5 à 5°C.

3. Procédure selon la revendication 2 dans laquelle le composé amorphe (1) de l'étape (iii) est lyophilisé à une température de -2 à 2°C.

4. Procédure selon la revendication 3 dans laquelle le composé (1) amorphe de l'étape (iii) est lyophilisé à une température de 0°C.

5. Formulation pharmaceutique comprenant la forme amorphe selon la revendication 1 et un ou plusieurs excipient(s) pharmaceutiquement acceptable(s).

6. Formulation de la revendication 5 sous la forme d'une crème, d'une lotion, d'un onguent et d'une solution pour pulvérisation.

7. Forme amorphe de la revendication 1 à utiliser dans le traitement ou la prévention contre des maladies ou des symptômes de la peau ou des membranes muqueuses.

8. Forme amorphe de la revendication 7 à utiliser dans le traitement ou la prévention contre les croûtes cutanées, la dermite atopique, la dermite de contact, la dermite séborrhéïque, la dermatose, l'eczéma, l'épidermolyse bulleuse simple, les érythèmes, les érosions, les squames, l'exsudation, l'inflammation, le lichen plan, le lichen rouge, la formation de papules, le prurit, l'érythème fessier, l'eczéma marginé de Hébra, le psoriasis ou les verrues.

9. Forme amorphe de la revendication 7 ou 8 qui est utilisée comme crème, lotion, onguent et solution pour pulvérisation.
